# EUROPEAN PATENT SPECIFICATION

(11) **EP 3 886 936 B1**
(45) Date of publication and mention of the grant of the patent: **09.09.2026**
(21) Application number: 20711333.3
(22) Date of filing: 03.03.2020
(51) Int. Cl.: A61M 1/06, A01J 5/007, A01J 5/10

(54) **BREASTPUMP WITH FUNCTIONAL VACUUM PULSES**
MILCHPUMPE MIT FUNKTIONELLEN VAKUUMIMPULSEN
TIRE-LAIT COMPRENANT DES IMPULSIONS DE VIDE FONCTIONNEL

(30) Priority: 07.03.2019 US 201962815135 P
(43) Date of publication of application: 06.10.2021
(73) Proprietor: Medela AG, 6340 Baar (CH)
(72) Inventor: STEVENS, Natalie, McHenry, Illinois 60050 (US); CHO, David Y., Arlington Heights, Illinois 60004 (US)
(74) Representative: Grünecker Patent- und Rechtsanwälte PartG mbB
(86) International application number: PCT/IB2020/051806
(87) International publication number: WO 2020/178737

(56) References cited:
- WO-A1-2020/051447
- JP-A- 2000 316 969
- JP-A- S5 796 659
- US-A- 5 902 267
- US-A1- 2009 254 029
- US-A1- 2019 046 700

## Description

### CROSS-REFERENCE TO RELATED APPLICATIONS

This is the non-provisional, and claims the benefit of the filing date under 35 USC § 119(e), of US Provisional Appl. No. 62/815,135, filed March 7, 2019.

### BACKGROUND

### Field of the Disclosure

This disclosure relates generally to expression of milk and, more specifically, to a breastpump that generates and delivers a pulsed vacuum.

### Description of the Prior Art

Breast alveoli share many functional similarities with lung alveoli. In the case of lung alveoli, oxygen and carbon dioxide are transferred between the lungs and the bloodstream, with carbon dioxide being transferred to the air and then expulsed. In the case of breast alveoli, components of breastmilk, such as nutrients, are transferred from the bloodstream to milk and then expulsed. A known treatment of occurrences of respiratory distress syndrome in full-term and near-term infants is the application of high-frequency oscillatory ventilation (HFOV). The efficacy of this form of ventilation is attributable to the fact that HFOV improves oxygenation through the lung alveoli. HFOV operates on a principle of vacuum pulse generation.

Conventional electric breastpumps deliver a cyclical vacuum, regardless of whether the breastpump is a single-stroke or accumulator style of breastpump. While strides have been made to provide breast pumps that apply forces at frequencies to the nipples that mimic those delivered by a feeding infants, the volume of milk expressed during pumping sessions utilizing conventional breastpumps can be lower than the volume of milk delivered by direct feeding. Moreover, while the fat content of milk provides some of the greatest nourishing benefits of breastfeeding, the concentration of fat within breast milk has been found to increase toward the end of a feeding or pumping session. High fat concentration milk can be the most desirable, because higher fat concentration in milk leads to a more complete removal of the milk to the feeding infant, or a more effective pump. The physiology of epithelial milk-secreting cells, however, is such that they tend to close-off as the fat concentration of the milk increases, thereby preventing the delivery of high fat concentration milk to the feeding infant or, in the case of a breastpump, to a milk collection container.

Relevant prior art is for instance disclosed in documents US2009/254029 A1, JP2000316969 A, JPS5796659 A, US5902267 A and US2019/046700 A1.

### Summary of the Disclosure

A breastpump according to the present invention comprises the technical features as defined in independent claim 1.

Given the functional similarities of breast alveoli and lung alveoli, we have designed a breast pump that utilizes vacuum pulse generation. By providing vacuum pulse generation in addition to (or in lieu of) a conventional cyclical vacuum, breastpumps of the present disclosure provide improved milk removal from the breast alveoli and foster the maintenance of open apertures of epithelial milk-secreting cells to provide improved milk removal from breast alveioli.

Advantageously, breastpumps of the present disclosure deliver higher volumes of collected breast milk in a single pumping session, while applying a lower effective vacuum pressure to the breast.

In a preferred embodiment, a breastpump employs a multi-stroke accumulator with inherent vacuum steps. While conventional accumulator pumps employ elastomeric diaphragms, such pumps experience losses due to the dampening effects the diaphragm and its related support structure have on the vacuum pulses generated by the motor of such pumps. Instead of an elastomeric diaphragm, the instant breastpump uses a solid piston head sealed in a cylinder. During use, the stiff piston creates a distinct vacuum step, or pulse, that can be transferred through vacuum delivery tubing to a breast shield or related device, across a media separation membrane (if necessary), and ultimately to the breast pumping mother.

Though constructed as a linear piston/cylinder-style breastpump, the system of the present disclosure takes advantage of the nature of a multi-stroke pump style in which a reciprocating member generates small increments of vacuum that are accumulated together to reach a target vacuum.

In order to deliver vacuum pulses with a solid piston, it is found beneficial to actuate the solid piston in an eccentric manner. Due to the need to maintain the integrity of a pneumatic seal between the circumference of the piston head and the walls of the vacuum cylinder despite the eccentric movement of the solid piston, a low friction compression seal having a non-circular cross-section configuration, such as the MULTISEAL^{®} bi-directional lip style seal available from Precision Associates, Inc. of Minneapolis, Minnesota, USA, is employed at the interface between the piston head and the sidewall of the vacuum cylinder.

The breastpump of the present disclosure applies a distinct vacuum pulse to stimulate the breast alveoli. The instant breastpump can achieve a higher vacuum than conventional diaphragm-style accumulator breastpumps operated under similar power conditions because energy losses attributable to material stretching are eliminated. Because a rigid piston can evacuate more volume in a chamber for each individual stroke, the breastpump of the present disclosure also can be constructed to have a smaller overall footprint than a diaphragm-style accumulator breastpump. Because diaphragm-style breastpumps are prone to fatigue, and even tears in the diaphragm membrane, the rigid piston-style pump of the present disclosure possesses superior reliability. Also, based on the strong correlation between the motor's current draw and the vacuum generated, vacuum control can be performed economically, without the need for costly pressure monitors. The mechanical architecture of a breastpump constructed in accordance with the teachings of the present disclosure is also tunable, in that the vacuum pulse frequency can be optimized by modifying the volume of the displacement chamber. The larger the chamber volume (which can be created either by using a larger cylinder cross-section or by increasing the piston stroke), the greater the vacuum step, and the smaller the chamber volume, the lower the vacuum step.

### DESCRIPTION OF THE SEVERAL VIEWS OF THE DRAWING

FIG. 1 is a semi-schematic view of a conventional diaphragm-style accumulator pump actuated by an eccentric connection to a pump motor;
FIG. 2 is a cross-sectional view of a multi-stroke accumulator linear piston/cylinder-style pump of the present disclosure, with an eccentric connection between the rigid piston and the motor shaft;

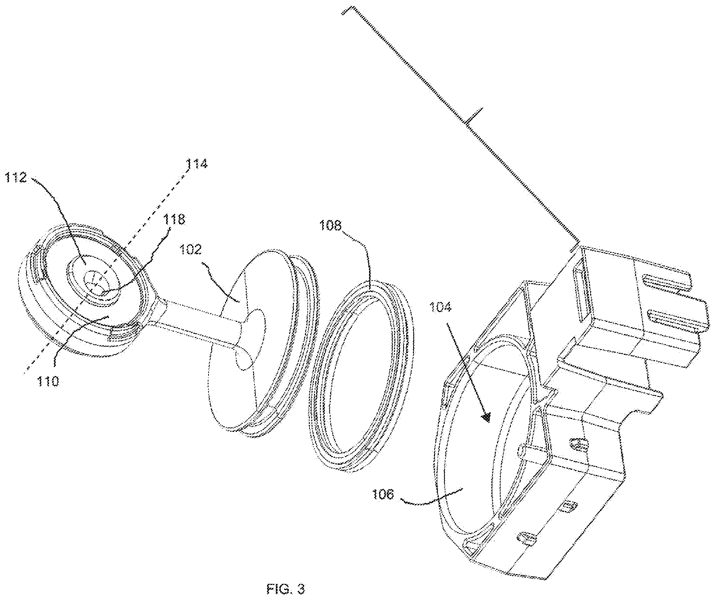

FIG. 3 is an exploded view of a rigid linear piston/cylinder and a multi-directional sealing gasket assembly of the pump of the present disclosure;

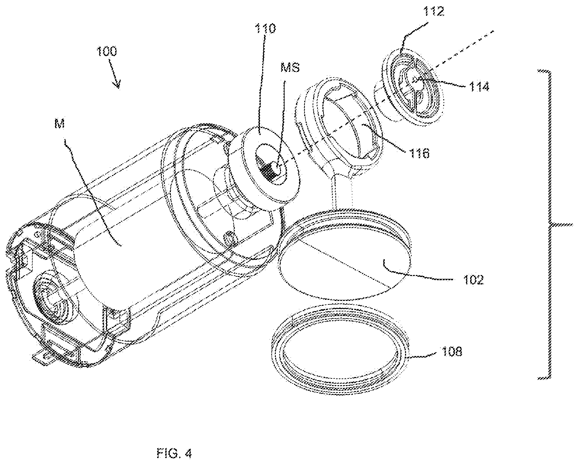

FIG. 4 is an exploded view of a rigid linear piston and eccentric roller bearing of the pump of the present disclosure, with a motor and a multi-directional sealing gasket;
FIG. 5 is an end view of a bearing insert of the linear piston of the pump of the present disclosure, with an offset-axis of the bearing insert in a first position, consistent with the piston's bottom-dead-center (or home) position;
FIG. 6 is an end view of the bearing insert of the linear piston of the pump of the present disclosure, with the offset-axis of the bearing insert in a second, intermediate position, consistent with the piston's eccentric (or tilted) position;
FIG. 7 is an end view of the bearing insert of the linear piston of the pump of the present disclosure, with the offset-axis of the bearing insert in a third position, consistent with the piston's top-dead-center (or chamber-evacuated) position;
FIG. 8 is a motor-end view of the bearing insert of the linear piston of the pump of the present disclosure, illustrating a shaft receptacle of the bearing insert in a first position, co-axial with the first position of the offset-axis of the bearing insert illustrated in FIG. 5, consistent with the piston's bottom-dead-center (or home) position;
FIG. 9 is a motor-end view of the bearing insert of the linear piston of the pump of the present disclosure, illustrating the shaft receptacle of the bearing insert in a second position, co-axial with the second position of the offset-axis of the bearing insert illustrated in FIG. 6, consistent with the piston's eccentric position;
FIG. 10 is a motor-end view of the bearing insert of the linear piston of the pump of the present disclosure, illustrating the shaft receptacle of the bearing insert in a third position, co-axial with the third position of the offset-axis of the bearing insert illustrated in FIG. 7, consistent with the piston's top-dead-center position;
FIG. 11 is an end view of a piston, bearing, bearing insert, and multi-directional gasket of the pump of the present disclosure, illustrating in solid lines the bearing insert in its first position and the piston in its corresponding bottom-dead-center (or home) position, and illustrating in phantom lines the second and third positions of the bearing insert (and respective corresponding eccentric and top-dead-center positions of the piston);
FIG. 12 is an end view of a piston, bearing, bearing insert, and multi-directional gasket similar to FIG. 11, but illustrating in solid lines the bearing insert in its second position and the piston in its corresponding eccentric position, and illustrating in phantom lines the first and third positions of the bearing insert (and respective corresponding bottom-dead-center (or home) and top-dead-center positions of the piston);
FIG. 13 is an end view of a piston, bearing, bearing insert, and multi-directional gasket similar to FIGS. 11 and 12, but illustrating in solid lines the bearing insert in its third position and the piston in its corresponding top-dead-center position, and illustrating in phantom lines the first and second positions of the bearing insert (and respective corresponding bottom-dead-center (or home) and eccentric positions of the piston);
FIG. 14 is an end view of a piston-cylinder assembly of the present disclosure, with the cylinder in phantom lines, the bearing insert in its second position, and the piston in its corresponding eccentric position;
FIG. 15 is a schematic representation of an exemplary cycle of the pump of the present disclosure;
FIG. 16 is a perspective view of a motor and piston-cylinder assembly of the pump of the present disclosure;
FIG. 17 is a cross-sectional view of the piston-cylinder assembly of the pump of the present disclosure, taken along lines 17-17 of FIG. 16, with the bearing insert in its second position and the piston in its corresponding eccentric position;

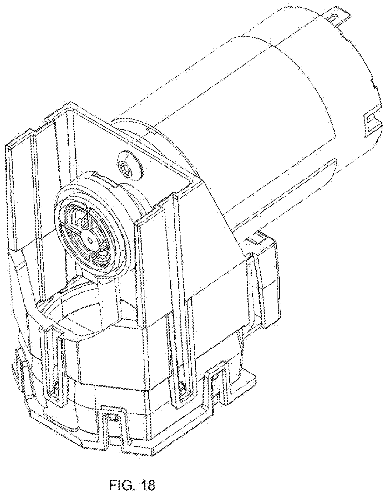

FIG. 18 is a perspective view of the pump of the present disclosure;

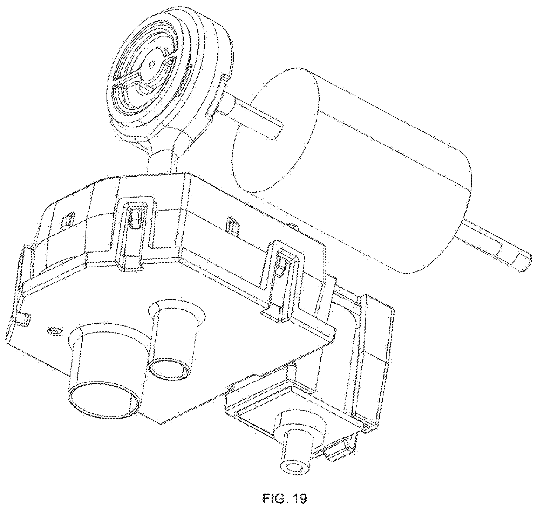

FIG. 19 is another perspective view of the pump of the present disclosure, with a housing of the motor removed;

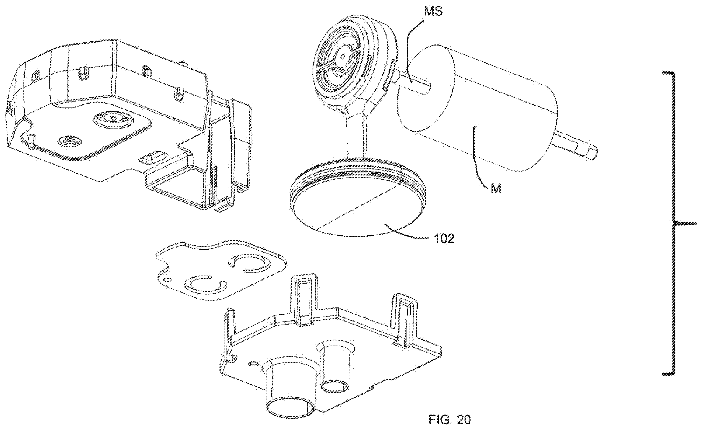

FIG. 20 is an exploded view of the motor and piston-cylinder assembly of the pump of the present disclosure;
FIG. 21 is a perspective view of the pump of the present disclosure, including a motor housing;
FIG. 22 is a graph of vacuum pressure vs. time (in units of mm Hg / seconds), comparing vacuum output from a conventional single-stroke pump, a conventional diaphragm pump, and a multi-stroke accumulator pump of the present disclosure;
FIG. 23 is a graph of milk flow rate (in units of grams/second) versus time (in minutes) of a pumping session utilizing a conventional accumulator breastpump (designated "18202 QUEEN" in Fig. 23) having four displacement chambers in multiple states of evacuation; and
FIG. 24 is a graph of milk flow rate (in units of grams/second) versus time (in minutes) of a pumping session utilizing a breastpump having a multi-stroke accumulator linear piston/cylinder-style pump of the present disclosure.

### DETAILED DESCRIPTION OF THE PREFERRED EMBODIMENTS

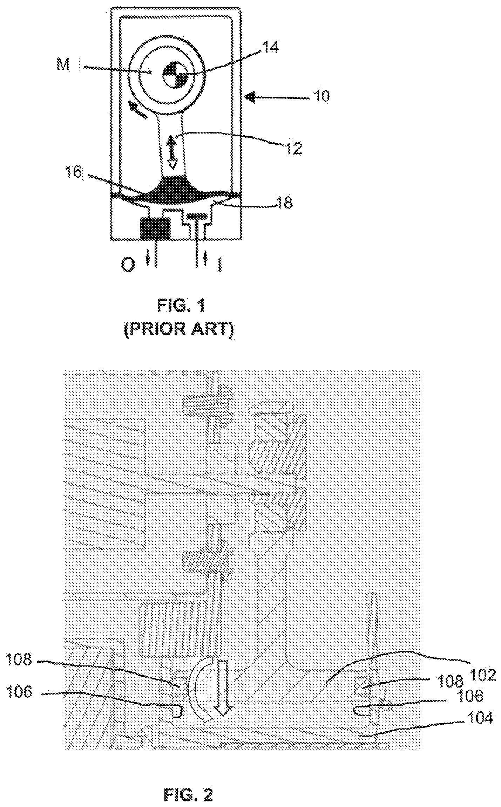

With reference to FIG. 1, a conventional diaphragm-style accumulator pump 10 is illustrated. A piston 12 of the diaphragm-style accumulator pump 10 has an eccentric engagement with an actuator motor (schematically represented in FIG. 1 by reference letter M) along a shaft 14. Through each revolution of the shaft 14, the piston 12 imparts first an up stroke, and next, a downstroke, to a diaphragm membrane 16. While this cyclical motion has the desired effect of building up pressure in a vacuum chamber 18, with each stroke, there are pressure losses across the membrane 16.

An accumulator-style pump 100 of the present disclosure is illustrated in cross-section in FIG. 2, and features a solid piston head 102 that is seated within a cylinder 104. The solid piston head 102 is actuated in an eccentric manner in order to deliver vacuum pulses to a surface to which vacuum pressure from the pulse is applied, such as a breast or a teat. The vacuum pressure may be applied directly, or to an intermediate surface such as a media separation membrane (not shown), used to maintain the integrity and sterility of the pump components and avoid contamination of collected fluid, such as breastmilk.

The solid piston head 102 engages a sidewall 106 of the cylinder 104. Because the solid piston head 102 is actuated in an eccentric manner, the piston head 102 does not maintain a perpendicular relationship with respect to the side wall 106 throughout its travel along the interior of the cylinder 104. Rather, the disc-like piston head 102 rocks from in a tilted manner as it moves up and down (as indicated by the curved and straight arrows in FIG. 2). In order to maintain sealed engagement of the piston head 102 with the sidewall 106 of the cylinder 104 throughout eccentric actuation of the piston head 102, it is preferred to use a multi-directional, low friction compression sealing gasket 108 having a non-circular cross-section configuration, such as the MULTISEAL^{®} bi-directional lip style seal available from Precision Associates, Inc. of Minneapolis, Minnesota, USA.

In a particular embodiment, the eccentric actuation of the solid piston head 102 can be achieved utilizing a rotatable bearing 110 that receives a bearing insert 112 having an offset axis of rotation 114. The rotatable bearing 110 and bearing insert 112 are received in a complementary circular opening 116 of the piston head 102. A motor M of the pump 100 includes a motor shaft MS that is securely received in a shaft receptacle 118 of the bearing insert 112, which shaft receptacle 118 is coaxial with the offset axis of rotation 114 of the bearing insert 112.

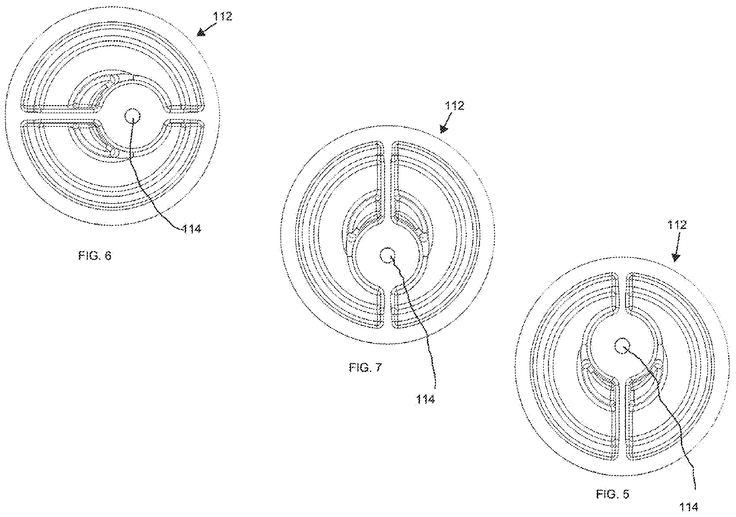

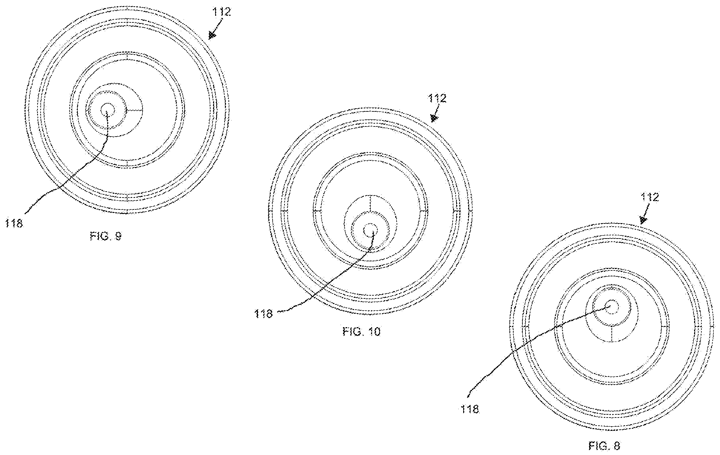

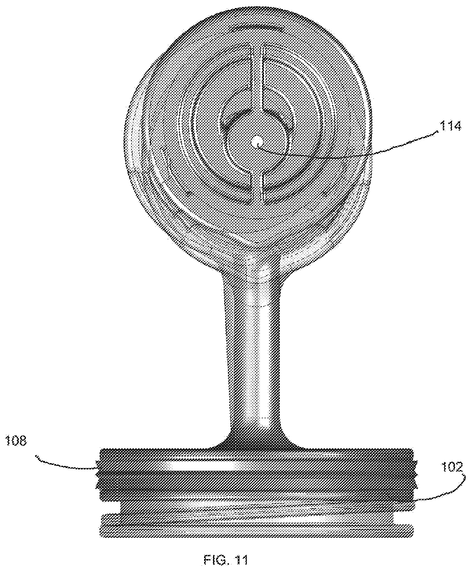

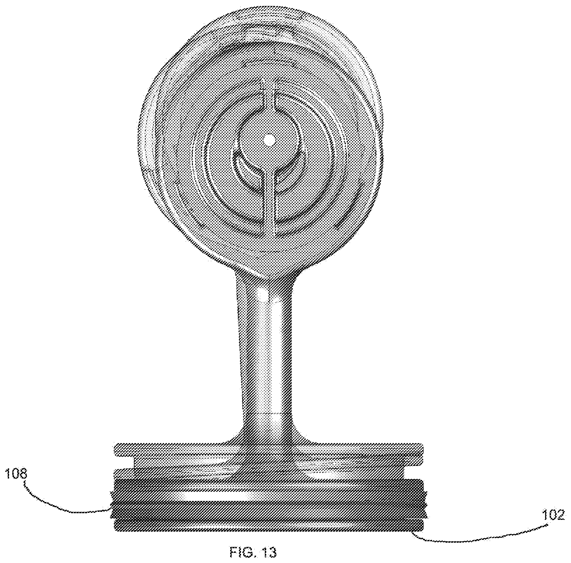

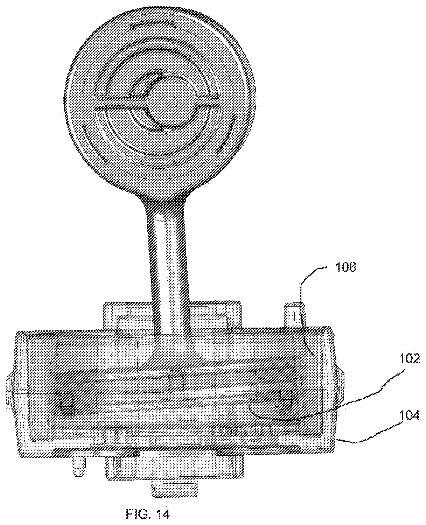

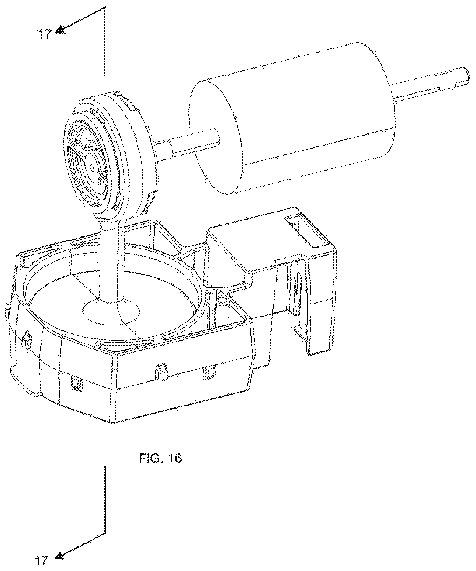

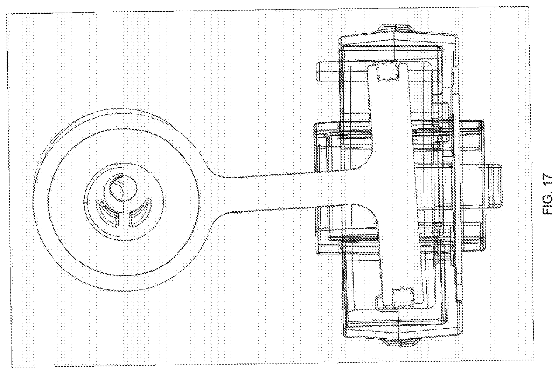

The sequence of actuation of the multi-stroke accumulator linear piston/cylinder-style pump of the present disclosure can be understood with reference to FIGS. 5-17. Three distinct positions of the bearing insert 112 are illustrated, respectively, in FIGS. 5, 6, and 7 (end views) and FIGS. 8, 9, and 10 (motor-end views). Corresponding views of the piston head 102 and sealing gasket 108 are illustrated in FIGS. 11, 12, and 13. FIGS. 5, 8, and 11 illustrate the bearing insert 112 in a first position, corresponding to a home or bottom-dead-center position of the piston head 102. FIGS. 6, 9, and 12 illustrate the bearing insert 112 in a second position, corresponding to an eccentric position of the piston head 102. FIGS. 7, 10, and 13 illustrate the bearing insert 112 in a third position, corresponding to a top-dead-center position of the piston head 102. While the piston head 102 and sealing gasket 108 are illustrated in solid lines in each of the various first, second, and third positions in FIGS. 11, 12, and 13, respectively, the alternate positions of the piston head 102 are illustrated in phantom lines in each of those figures.

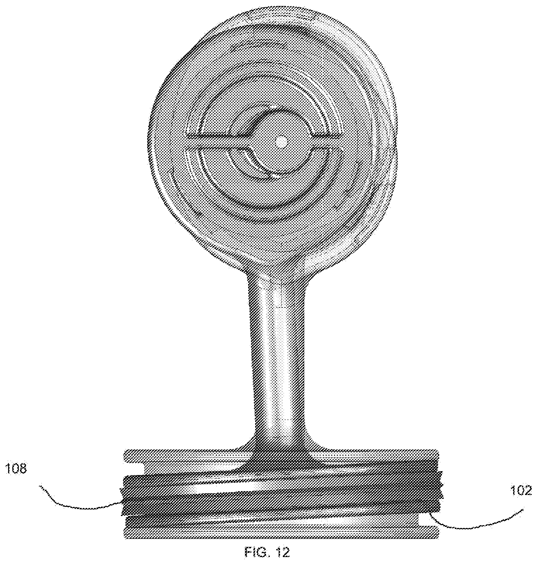

With reference to FIGS. 12, 14, and 17, it can be appreciated that when the piston head 102 is in the eccentric position, the piston head 102 has moved not only vertically, but also to an angle relative to the sidewall 106 of the cylinder 104. As compared to conventional single-stroke pistons, the pump 100 of the present disclosure advantageously applies a vacuum pulse frequency over the cyclical vacuum frequency that would otherwise be applied had the piston head 102 only moved up and down once. The vacuum pulse can be attributed to the multiple rotations (approximately 20) of the piston head 102 relative to the sidewall 106 of the cylinder 104 during the cyclical vacuum cycle. Two check valves allow the incremental building of vacuum with each rotation until the target vacuum is achieved. The volume of air displaced during a single rotation of the piston is approximately 1/20^{th} of the volume of air displaced for a conventional single-stroke piston.

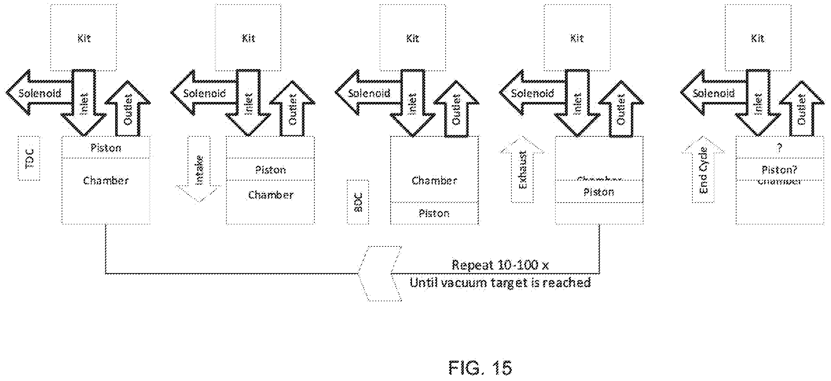

An exemplary cycle of the pump of the present disclosure is illustrated in FIG. 15. The following table indicates the status of each component of the cycle at a given state of the cycle:

| **Ref.** | **State** | **Piston** | **Inlet** | **Outlet** | **Solenoid** |
|---|---|---|---|---|---|
| 1 | Top Dead Center (TDC) | 0 | 1 | 0 | 0 |
| 2 | Intake | 1 | 1 | 0 | 0 |
| 3 | Bottom Dead Center (BDC) | 0 | 1 | 0 | 0 |
| 4 | Exhaust | 1 | 0 | 1 | 0 |
| 5 | End Cycle | 1 | 0 | 1 | 1 |

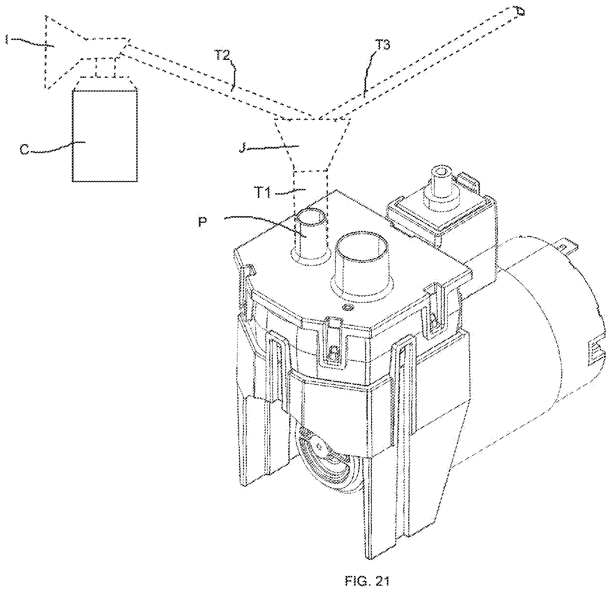

As illustrated in FIG. 21, the pump includes a port P that may be placed into fluid communication with a tubing system (illustrated in broken lines in the figure), including a first length of tubing T1, a junction J, a second length of tubing T2 that is in fluid communication with a first breastmilk collection kit K including a milk collection container C and an interface I, such as a breastshield, that receives the breast (not shown) and a media separation membrane (also not shown) to permit vacuum to be applied to the interior of the interface, and thus to the breast received therein, and permit collection of breastmilk, while isolating the tubing circuit from the breastmilk, thereby preventing contamination of the pump. The system can optionally further include, in order to provide simultaneous pumping, a third length of tubing T3 that is in fluid communication with a second breastmilk collection kit (not shown).

Other embodiments that are within the scope of the present disclosure include alternate structural configurations for a vacuum pump mechanism that achieve controllable vibration pulses. For instance, rather than having a piston that undergoes an eccentric movement, a vacuum pump having a piston that maintains a vertical orientation throughout its travel along the sidewall 106 of the cylinder 104, but employs a yoke, linkages, and/or direct action (such as a solenoid, a servo motor, or a DC stepper motor capable of pulsed actuation).

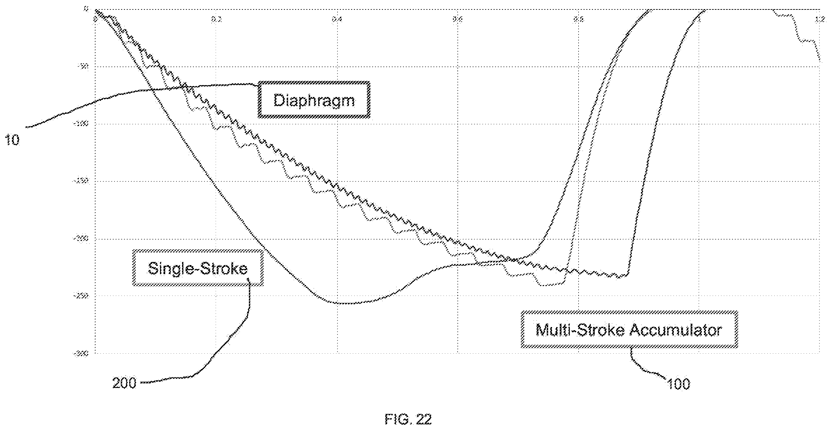

FIG. 22 graphically compares vacuum-versus-time plots of a conventional single-stroke pump 200 (which, as can be seen in the figure, generates a cyclical vacuum reflected by a smooth-lined vacuum curve), a conventional diaphragm-style accumulator pump 10, and a multi-stroke accumulator pump 100 of the present disclosure. While a diaphragm-style accumulator pump 10 can apply a non-smooth vacuum curve, the amplitude of the variations in vacuum pressure are de minimis, likely due to the dampening effect of the diaphragm membrane 16. Moreover, due to the deterioration of the diaphragm membrane 16 over time, such as due to fatigue, cracking, or tearing of the membrane 16, vacuum curves of a diaphragm-style accumulator pump 10 tend to smooth out over time. There is also a lack of precision and fundamental difficulty in the ability to repeatedly obtain predictable vacuum pressure variation with a diaphragm-style accumulator pump 10. The multi-stroke accumulator pump 100, by virtue of the eccentric movement of its rigid piston head 102 and lack of a diaphragm membrane 16, has the ability to be reliably tuned to a predictable vacuum frequency.

The motor M of the multi-stroke accumulator pump 100 of the present disclosure has an increased torque relative to motors employed with a conventional single-stroke pump 200 or a diaphragm-style accumulator pump 10, which is necessary to achieve the discernible, predictable vacuum pulses of sufficient amplitude to achieve advantageous results, as described in more detail below. A benefit of the increased torque of the motor M is that each vacuum pulse creates a distinct load on the motor shaft MS that is directly proportional to the current draw of the motor M. As such, the vacuum load can be accurately predicted based on the current draw.

Another benefit of the correlation between the current draw of the motor M and the resulting vacuum of the multi-stroke accumulator pump 100 of the present disclosure is that the pump 100 can be controlled by monitoring the current output of the motor M without the need to include a pressure monitor. To avoid applying too strong of a vacuum, a predetermined not-to-exceed current output of the motor M can be set.

A practical effect of the predictable vacuum pulse generated by the multi-stroke accumulator pump 100 of the present disclosure when employed in a breast pump is increased oxygenation of breast alveoli and improved milk removal, as compared to vacuum supplied by conventional single-stroke pumps 200 or diaphragm-style accumulator pumps 10. The combined cyclical vacuum frequency and vacuum pulse frequency applied by the multi-stroke accumulator pump 100 imparts a high-frequency oscillatory ventilation to the breast alveoli. In a manner similar to the treatment of respiratory distress syndrome through the application of HVOC, which utilizes vacuum pulse generation to provide increased oxygenation to lung alveoli, increased stimulation of the breast alveoli during the course of a breast pumping session is found to result in an increased volume of collected breastmilk.

Percent of available milk removed, or PAMR, is a metric used to quantify the volume of collected breastmilk.

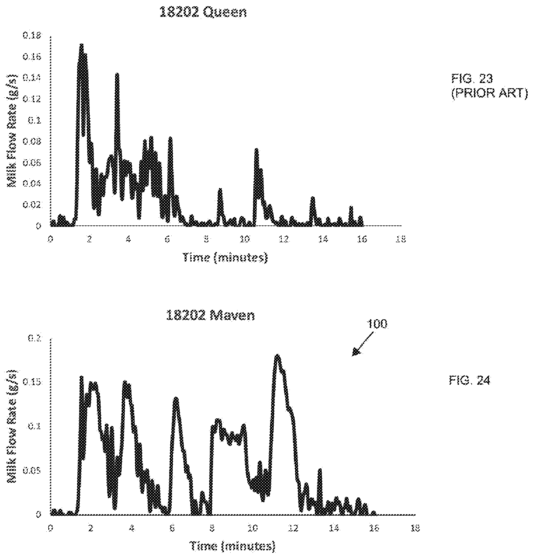

A comparison of FIGS. 23 and 24 graphically illustrates a measurable increase in milk flow rate during the course of a pumping session using a multi-stroke accumulator pump 100 of the present disclosure (FIG. 24), as compared to milk flow rate during the course of a pumping session by the same nursing mother using a conventional accumulator breastpump (FIG. 23).

Moreover, it is understood that breastmilk having higher fat concentration leads to more complete removal of the milk to a feeding infant, and a more effective pump. During the course of a pumping session, it is found that the viscosity of breastmilk increases toward the end of the pumping session, indicative of an increase in fat content of the breastmilk. However, with conventional cyclical vacuum breast pumps, the less-ventilated breast alveoli tend to close up, or the viscosity of the breastmilk otherwise impedes the secretion of further breastmilk through the breast alveoli, despite the epithelial milk-secreting cells having generated additional desirable high-nutrient, relatively high-fat breastmilk. This precious additional breastmilk, when not released through the breast alveoli, goes unused. Over time, the inability to remove the all available milk from the breast alveoli, whether through baby feeding, pumping, or some combination of the two, will diminish available milk supply. Thus, an advantage of the multi-stroke accumulator pump 100 of the present disclosure, and other vacuum pump mechanisms that achieve controllable vibration pulses which are within the scope of the present disclosure, is the provision of a device that facilitates complete or near-complete emptying of the breast alveoli, which promotes increased, or at least sustained, milk supply over time. Through the use of the multi-stroke accumulator pump 100 of the present disclosure, it is found that not only is increased breastmilk collection during a given pumping cycle achieved, but also, the collected breastmilk includes the higher fat content breastmilk that heretofore went uncollected by electric and manual breastpumps.

Yet a further benefit of the multi-stroke accumulator pump 100 of the present disclosure, and other vacuum pump mechanisms that achieve controllable vibration pulses which are within the scope of the present disclosure, is that the increase in milk flow rate during the course of a pumping session is achieved via application of less vacuum pressure. Practical benefits of being able to achieve increased milk collection with lower vacuum pressure include lower power requirements (facilitating use of less-costly power supplies, lighter power supplies, and/or rechargeable power supplies that require less time to re-charge or that maintain their charge longer before they require a recharging), and greater comfort for the nursing mother. The increased pumping efficiency also affords several benefits, such as faster pumping (and therefore shorter pumping sessions), greater breastmilk collection in a single pumping session, and, as discussed above, increased, or at least less-diminished, milk supply over time.

While the present disclosure is presented with human breastpumping as its primary use, milking machines for use in the dairy industry may be constructed with pumping mechanisms that utilize a multi-stroke accumulator pump made according to the teachings of this disclosure, and other vacuum pump mechanisms that achieve controllable vibration pulses, to achieve many, if not all, of the benefits described herein. For milking machines in the dairy industry, for instance, the interface I may instead be one that receives one or more bovine teats. A single pump of the present disclosure could be used in such settings in simultaneous pneumatic communication with more than two teats, and even with more than a single lactating cow, so as to milk a plurality of cows simultaneously.

A multi-stroke accumulator pump 100 may be operated at 0.1mmHg or higher, and a frequency below 80 Hz. The frequency can be below 30 Hz, or in the range of 10-30 Hz.

While certain embodiments are described herein, the scope of the present invention is defined by the appended claims.

## Claims

1. A breastpump comprising:
a vacuum source that is configured to apply a cyclical vacuum, the vacuum source including an accumulator-style pump (100), with a drive motor (M) having a motor shaft (MS) and a rigid, disc-like piston head (102) configured to be actuated by the motor in an eccentric manner, seated in a cylinder (104) and engaged with and sealed against a sidewall (106) of the cylinder (104), **characterized in that** the piston head (102) is configured to be actuated so that the piston head (102) does not maintain a perpendicular relationship to the sidewall (106) of the cylinder (104) during its travel within the cylinder (104), such that the piston head (102) rocks in a tilted manner as it moves up and down relative to the cylinder (104) while maintaining a sealed engagement with the sidewall (106) of the cylinder (104) and that the vacuum source also applies a vacuum pulse frequency.

2. The breastpump of claim 1, **characterized in that** it further comprises:
one or more valves provided on a wall of the cylinder (104), the one or more valves permitting application of negative pressure that accumulates in the cylinder (104) after a plurality of cycles of the piston head (102) in the cylinder (104).

3. The breastpump of claim 2, **characterized in that** each vacuum pulse is configured to create a distinct load on the motor shaft (MS) that is directly proportional to a current draw of the motor.

4. The breastpump of claim 3, **characterized in that** it further comprises a sensor that senses the effect on the current draw of the motor by the load applied to the motor shaft (MS) by each vacuum pulse, and a controller, the controller including a memory stored on a non-transitory computer readable medium that includes a target vacuum pressure, and a comparator that compares the sensed effect on the current draw of the motor from the sensor to the stored target vacuum pressure, and selectively opens or closes the one or more valves based on whether the comparator determines the effect on the current draw of the motor is above, below, or equal to, the target vacuum pressure.

5. The breastpump of claim 2, **characterized in that** it is in further combination with at least one breastshield and tubing in pneumatic communication with the breastshield and one or more valves of the cylinder (104).

## Patentansprüche

1. Milchpumpe, die umfasst:
eine Vakuumquelle, die so ausgeführt ist, dass sie ein zyklisches Vakuum erzeugt, wobei die Vakuumquelle eine Speichertyp-Pumpe (100) mit einem Antriebsmotor (M), der eine Motorwelle (MS) aufweist, und einem starren scheibenartigen Kolbenkopf (102) einschließt, der so ausgeführt ist, dass er von dem Motor exzentrisch betätigt wird, in einem Zylinder (104) aufgenommen und mit einer Seitenwand (106) des Zylinders (104) in Kontakt ist und daran abdichtet,
**dadurch gekennzeichnet, dass**
der Kolbenkopf (102) so ausgeführt ist, dass er so betätigt wird, dass der Kolbenkopf (102) während seiner Bewegung im Inneren des Zylinders (104) keine senkrechte Beziehung zu der Seitenwand (106) des Zylinders (104) aufrechterhält, sodass der Kolbenkopf (102) schräg geschwenkt wird, wenn er sich relativ zu dem Zylinder (104) nach oben und nach unten bewegt, und dabei einen abgedichteten Kontakt mit der Seitenwand (106) des Zylinders (104) aufrechterhält, und dass die Vakuumquelle auch eine Vakuumpuls-Frequenz zur Anwendung bringt.

2. Milchpumpe nach Anspruch 1, **dadurch gekennzeichnet, dass** sie des Weiteren umfasst:
ein oder mehr Ventil/e, das/die an einer Wand des Zylinders (104) vorhanden ist/sind, wobei das eine oder die mehr Ventil/e Ausübung eines Unterdrucks ermöglichen, der sich in dem Zylinder (104) nach einer Vielzahl von Zyklen des Kolbenkopfes (102) in dem Zylinder (104) akkumuliert.

3. Milchpumpe nach Anspruch 2, **dadurch gekennzeichnet, dass** jeder Vakuumpuls so ausgeführt ist, dass er eine bestimmte Last auf die Motorwelle (MS) ausübt, die direkt proportional zu einer Stromaufnahme des Motors ist.

4. Milchpumpe nach Anspruch 3, **dadurch gekennzeichnet, dass** sie des Weiteren einen Sensor umfasst, der den Effekt auf die Stromaufnahme des Motors durch die auf die Motorwelle (MS) mit jedem Vakuumpuls ausgeübte Last erfasst, und eine Steuerungseinrichtung, wobei die Steuerungseinrichtung einen Speicher einschließt, der auf einem nichtflüchtigen computerlesbaren Speichermedium aufgezeichnet ist und einen Vakuum-Druck einschließt, sowie eine Vergleichseinrichtung umfasst, die den erfassten Effekt auf die Stromaufnahme des Motors von dem Sensor mit dem gespeicherten Soll-Vakuumdruck vergleicht, und dass eine oder die mehr Ventil/e basierend darauf selektiv öffnet oder schließt, ob die Vergleichseinrichtung feststellt, dass der Effekt auf die Stromaufnahme des Motors über, unter oder auf dem Soll-Vakuumdruck liegt.

5. Milchpumpe nach Anspruch 2, **dadurch gekennzeichnet, dass** sie des Weiteren eine Kombination mit wenigstens einer Brusthaube und einem Schlauch bildet, der in pneumatischer Verbindung mit der Brusthaube und einem oder mehr Ventile/n des Zylinders (104) steht.

## Revendications

1. Tire-lait comprenant :
une source de vide qui est configurée pour appliquer un vide cyclique, la source de vide comportant une pompe de type accumulateur (100), avec un moteur d'entraînement (M) ayant un arbre de moteur (MS) et une tête de piston rigide en forme de disque (102) configurée pour être actionnée par le moteur d'une manière excentrique, assise dans un cylindre (104) et en prise avec une paroi latérale (106) du cylindre (104), et scellée contre celle-ci,
**caractérisé en ce que** la tête de piston (102) est configurée pour être actionnée de sorte que la tête du piston (102) ne maintient pas une relation perpendiculaire avec la paroi latérale (106) du cylindre (104) pendant son déplacement à l'intérieur du cylindre (104), de sorte que la tête de piston (102) bascule d'une manière inclinée lorsqu'elle se déplace vers le haut et le bas par rapport au cylindre (104) tout en maintenant une mise en prise scellée avec la paroi latérale (106) du cylindre (104) et **en ce que** la source de vide applique également une fréquence d'impulsion de vide.

2. Tire-lait selon la revendication 1, **caractérisé en ce qu'**il comprend en outre :
une ou plusieurs soupapes agencées sur une paroi du cylindre (104), les une ou plusieurs soupapes permettant l'application d'une pression négative qui s'accumule dans le cylindre (104) après une pluralité de cycles de la tête de piston (102) dans le cylindre (104).

3. Tire-lait selon la revendication 2, **caractérisé en ce que** chaque impulsion de vide est configurée pour créer une charge distincte sur l'arbre de moteur (MS) qui est directement proportionnelle à une consommation de courant du moteur.

4. Tire-lait selon la revendication 3, **caractérisé en ce qu'**il comprend en outre un capteur qui détecte l'effet sur la consommation de courant du moteur par la charge appliquée à l'arbre de moteur (MS) par chaque impulsion de vide, et un dispositif de commande, le dispositif de commande comportant une mémoire stockée sur un support non transitoire lisible par ordinateur qui comporte une pression de vide cible, et un comparateur qui compare l'effet détecté sur la consommation de courant du moteur provenant du capteur à la pression de vide cible stockée, et ouvre ou ferme sélectivement les une ou plusieurs soupapes selon que le comparateur détermine que l'effet sur la consommation de courant du moteur est supérieure, inférieure ou égale à la pression de vide cible.

5. Tire-lait selon la revendication 2, **caractérisé en ce qu'**il est en outre en combinaison avec au moins une téterelle et un tubage en communication pneumatique avec la téterelle et une ou plusieurs soupapes du cylindre (104).
